# EUROPÄISCHE PATENTSCHRIFT

(11) **EP 3 574 890 B1**
(45) Veröffentlichungstag und Bekanntmachung des Hinweises auf die Patenterteilung: **07.06.2023**
(21) Anmeldenummer: 19176672.4
(22) Anmeldetag: 27.05.2019
(51) Int. Cl.: A61K 8/44, A61K 8/67, A61K 8/55, A61K 8/92, A61K 8/9789, A61K 36/185, A61Q 7/00, A61P 17/04, A61P 17/14, A61K 31/197, A61K 31/685, A61K 36/258, A61K 36/28, A61K 36/53, A61K 36/61, A61K 36/82, A61K 36/9066

(54) **FLÜSSIGES STOFFGEMISCH ZUM AUFTRAGEN AUF DIE KOPFHAUT**
LIQUID MIXTURE FOR APPLICATION ON THE SKIN OF THE HEAD
MÉLANGE DE MATIÈRE LIQUIDE DESTINÉ À L'APPLICATION SUR LE CUIR CHEVELU

(30) Priorität: 29.05.2018 DE 102018112826
(43) Veröffentlichungstag der Anmeldung: 04.12.2019
(73) Patentinhaber: BUK Management GmbH, 01067 Dresden (DE)
(72) Erfinder: Brockmann, Petra, 01324 Dresden (DE)
(74) Vertreter: Kirchner, Veit

(56) Entgegenhaltungen:
- DE-U1- 20 315 174
- US-A1- 2002 182 196
- US-A1- 2012 189 674
- DATABASE GNPD [Online] MINTEL; 15. August 2006 (2006-08-15), anonymous: "Lavender & Anthyllis Leave-In Conditioner", XP055632904, gefunden im www.gnpd.com Database accession no. 574410

## Beschreibung

Die Erfindung betrifft die nicht-therapeutische Verwendung eines flüssiges Stoffgemisch zum Auftragen auf die Kopfhaut.

Unterschiedliche Pflegeprodukte zum Auftragen auf die Kopfhaut sind bekannt. Diese umfassen unter anderem reinigende Haarshampoos sowie pflegende Conditioner und Haarwasser (Tonika). Neben einem reinigenden und allgemein pflegenden Effekt werden von Verbrauchern zudem auch naturbelassene Inhaltsstoffe und Aromen nachgefragt. Speziell bei männlichen Kunden ist oft auch eine Stimulierung des Haarwachstums gewünscht. Generell sollen die Produkte eine pflegende Wirkung auf die Kopfhaut haben. So beschreiben US 2012/0189674 A1 und DE 203 15 174 U1 Mittel mit Gehalten an Aminosäuren und Sandelholzöl und haarwuchsfördernder Wirkung.

Aufgabe der Erfindung ist es, eine Zusammensetzung zum Auftragen auf die Kopfhaut bereitzustellen, die natürliche Wirkstoffe enthält und mit der eine Harmonisierung und Verbesserung des Kopfhautzustandes und damit des Haarwachstums erreicht werden kann.

Vor diesem Hintergrund betrifft die Erfindung die nicht-therapeutische Verwendung eines flüssigen Stoffgemischs zum Auftragen auf die Kopfhaut umfassend wenigstens eine Aminosäure, Biotin als B-Vitamin, Lecithin sowie Sandelholzöl.

Das Stoffgemisch umfasst somit einen Wirkstoffkomplex aus mehreren natürlichen Wirkstoffen. Die Zusammensetzung des Wirkstoffkomplexes vereint ayurvedische und europäische Heilkunde mit modernen wissenschaftlichen Erkenntnissen und wirkt harmonisierend und pflegend auf die Kopfhaut sowie stimulierend auf das Haarwachstum. So kann durch die beanspruchte Wirkstoffkombination eine signifikante Verbesserung des Kopfhautzustandes und des Haarwachstums erreicht werden. Die Pflanzenextrakte sorgen zudem für einen angenehmen natürlichen Duft. Nachfolgend werden besonders bevorzugte Ausgestaltungen des Wirkstoffkomplexes aus Aminosäuren, Biotin, Sandelholzöl und Lecithin beschrieben.

In einer Ausführungsform umfasst das Stoffgemisch Arginin als Aminosäure. Das Arginin ist vorzugsweise in einer Menge von zwischen 0,5 und 1,5 Gew.-% im Stoffgemisch enthalten.

In einer Ausführungsform umfasst das Stoffgemisch Lysin als Aminosäure. Das Lysin ist vorzugsweise in einer Menge von zwischen 0,1 und 0,4 Gew.-% im Stoffgemisch enthalten.

In einer Ausführungsform umfasst das Stoffgemisch Calciumpantothenat als weiteres B-Vitamin. Das Calciumpantothenat ist vorzugsweise in einer Menge von zwischen 0,1 und 0,5 Gew.-% im Stoffgemisch enthalten.

Das Stoffgemisch umfasst Biotin als B-Vitamin. Das Biotin ist vorzugsweise in einer Menge von zwischen 0,001 und 0,01 Gew.-% im Stoffgemisch enthalten.

Das Stoffgemisch umfasst Lecithin. Das Lecithin ist vorzugsweise in einer Menge von zwischen 1 und 2 Gew.-% im Stoffgemisch enthalten.

Das Stoffgemisch umfasst sogenanntes Sandelholzöl, also ein Extrakt des Sandelholzbaums (Santalum album) als Pflanzenextrakt. Es ist bekannt, dass bestimmte Hautzellen, die Keratinozyten, den Duftrezeptor OR2AT4 besitzen. Dieser Rezeptor wird durch Duftstoffe mit einer Sandelholznote aktiviert, wodurch die Hautregeneration und Wundheilung maßgeblich gesteigert werden können. Die Erfindung macht sich im vorliegenden Kontext nun zu Nutze, dass der Duftrezeptor OR2AT4 sich auch in den sogenannten Matrixzellen der Haarwurzel findet, die für das Wachstum verantwortlich sind.

In einer Ausführungsform umfasst das Stoffgemisch Ginseng-Extrakt als Pflanzenextrakt. Das Ginseng-Extrakt ist vorzugsweise in einer Menge von zwischen 0,2 und 0,7 Gew.-% im Stoffgemisch enthalten.

In einer Ausführungsform umfasst das Stoffgemisch Extrakt von grünem Tee als Pflanzenextrakt. Das Extrakt von grünem Tee ist vorzugsweise in einer Menge von zwischen 0,2 und 0,7 Gew.-% im Stoffgemisch enthalten.

In einer Ausführungsform umfasst das Stoffgemisch Hamamelis-Extrakt als Pflanzenextrakt. Das Hamamelis-Extrakt ist vorzugsweise in einer Menge von zwischen 0,2 und 0,7 Gew.-% im Stoffgemisch enthalten.

In einer Ausführungsform umfasst das Stoffgemisch Kamillen-Extrakt als Pflanzenextrakt. Das Kamillen-Extrakt ist vorzugsweise in einer Menge von zwischen 0,2 und 0,7 Gew.-% im Stoffgemisch enthalten.

In einer Ausführungsform umfasst das Stoffgemisch Kurkuma-Extrakt als Pflanzenextrakt. Das Kurkuma-Extrakt ist vorzugsweise in einer Menge von zwischen 0,2 und 0,7 Gew.-% im Stoffgemisch enthalten.

In einer Ausführungsform umfasst das Stoffgemisch Teebaum-Extrakt als Pflanzenextrakt. Das Teebaum-Extrakt ist vorzugsweise in einer Menge von zwischen 0,2 und 0,7 Gew.-% im Stoffgemisch enthalten.

In einer Ausführungsform umfasst das Stoffgemisch Basilikum-Extrakt als Pflanzenextrakt. Das Basilikum-Extrakt ist vorzugsweise in einer Menge von zwischen 0,2 und 0,7 Gew.-% im Stoffgemisch enthalten.

Alle genannten Angaben in Gew.-% verstehen sich als bezogen auf die Gesamtmasse des Stoffgemischs. Allgemein ist der bevorzugte Wirkstoffgehalt hoch, sodass anhand des erfindungsgemäßen Stoffgemischs eine signifikante Wirkung bei Haut und Haar erreicht werden kann.

Neben den genannten Wirkstoffen umfasst das Stoffgemisch typischerweise ein Lösungsmittel, vorzugsweise Wasser, mit einem Anteil von mehr als 60 Gew.-%, vorzugsweise mehr als 75 Gew.-%.

In einer Ausführungsform handelt es sich bei dem Stoffgemisch um ein Haarwasser. Das Haarwasser bzw. Tonikum umfasst vorzugsweise die Stoffe in wässriger, gemischt wässrig-alkoholischer oder alkoholischer Lösung. Um ein möglichst naturnahes Produkt bereitzustellen wird vorzugsweise auf Parfum und Farbstoffe verzichtet. In einer Ausführungsform besteht das Haarwasser aus dem Lösungsmittel, den genannten Aminosäuren, B-Vitaminen und Pflanzenextrakten sowie dem Lecithin.

Die Erfindung betrifft die nicht-therapeutische Verwendung eines erfindungsgemäßen Stoffgemischs zum Auftragen auf die Kopfhaut und/oder Haare. Grundsätzlich eignet sich das erfindungsgemäße Stoffgemisch dabei für jeden Haut- und Haartyp sowie jeden Haut- und Haarzustand. Das Stoffgemisch ist zur Verwendung bei Männern und Frauen jeglichen Alters geeignet.

Weitere Einzelheiten der Erfindung ergeben sich aus den nachfolgend beschriebenen Ausführungsbeispielen.

### Beispiel 1:

Zur Herstellung eines erfindungsgemäßen Haarwassers werden die folgenden Bestandteile bei Raumtemperatur in einen Behälter gegeben und bis zur Homogenität mit einem Lösungsmittel aus ca 90/10 (v/v) Wasser/Ethanol verrührt (Gewichtsangaben basierend auf dem Gesamtgemisch inkl. Lösungsmittel):
1,5 Gew.-% Arginin
0,4 Gew.-% Lysin
0,4 Gew.-% Calciumpantothenat
0,01 Gew.-% Biotin
1,5 Gew.-% Lecithin
0,6 Gew.-% Ginseng-Extrakt
0,6 Gew.-% Extrakt von grünem Tee
0,6 Gew.-% Hamamelis-Extrakt
0,6 Gew.-% Kamillen-Extrakt
0,2 Gew.-% Kurkuma-Extrakt
0,2 Gew.-% Teebaum-Extrakt
0,6 Gew.-% Basilikum-Extrakt
0,1 Gew.-% Sandelholzöl

### Probandenstudie:

Im Rahmen einer Probandenstudie wurde das Haarwasser aus Beispiel 1 an 80 Probanden getestet. Dabei konnten die folgenden Aussagen festgehalten werden.
a. 50 von 80 Verwendern (62,5%) würden das Produkt weiterempfehlen.
b. 45 von 80 Verwendern (56,25%) sagen aus, dass sie viele, kleine nachwachsende Haare spüren.
c. 46 von 80 Verwendern (57,5%) sagen aus, dass sich ihre Haare viel voller anfühlen.
d. 42 von 80 Verwendern (52,5%) sagen aus, dass sich ihre Haare insgesamt kräftiger anfühlen.
e. 42 von 80 Verwendern (52,5%) sagen aus, dass ihr Haarausfall weniger geworden ist.
f. 30 von 80 Verwendern (37,5%) geben an, dass ihre Kopfhaut beruhigter ist.
g. 29 von 80 Verwendern (36,25%) sagen aus, dass sie weniger Juckreiz auf ihrer Kopfhaut spüren.
h. 20 von 80 Verwendern (25%) sagen aus, dass sie weniger Rötungen auf der Kopfhaut haben.
i. 18 von 80 Verwendern (22,5%) geben an, dass sie weniger Schuppen haben.
j. 25 von 80 Verwendern (31,25%) geben an, dass sie weniger Spannungsgefühl auf der Kopfhaut haben.

Zu diesen Ergebnissen ist noch anzumerken, dass bei den Kriterien f.-i. nicht jeder der 80 Probanden die zu bewertenden Beschwerden (juckende Kopfhaut, Rötungen oder Schuppen) hatte. Daher konnte bei diesen Kriterien nicht jeder Proband eine Angabe machen, sodass die tatsächlichen Prozentzahlen in diesen Fällen jedenfalls größer sind, als die angegebenen Prozentzahlen.

## Patentansprüche

1. Nicht-therapeutische Verwendung eines flüssigen Stoffgemischs zum Auftragen auf die Kopfhaut, wobei das Stoffgemisch wenigstens die folgenden Inhaltsstoffe umfasst:
eine Aminosäure,
Biotin,
Lecithin,
Sandelholzöl.

2. Verwendung nach Anspruch 1, **dadurch gekennzeichnet, dass** das Stoffgemisch Arginin als Aminosäure umfasst, wobei das Arginin vorzugsweise in einer Menge von zwischen 0,5 und 1,5 Gew.-% gemessen an der Gesamtmasse des Stoffgemischs enthalten ist.

3. Verwendung nach einem der vorhergehenden Ansprüche, **dadurch gekennzeichnet, dass** das Stoffgemisch Lysin als Aminosäure umfasst, wobei das Lysin vorzugsweise in einer Menge von zwischen 0,1 und 0,4 Gew.-% gemessen an der Gesamtmasse des Stoffgemischs enthalten ist.

4. Verwendung nach einem der vorhergehenden Ansprüche, **dadurch gekennzeichnet, dass** das Stoffgemisch weiterhin Calciumpantothenat umfasst, wobei das Calciumpantothenat vorzugsweise in einer Menge von zwischen 0,1 und 0,5 Gew.-% gemessen an der Gesamtmasse des Stoffgemischs enthalten ist.

5. Verwendung nach einem der vorhergehenden Ansprüche, **dadurch gekennzeichnet, dass** das Biotin in einer Menge von zwischen 0,001 und 0,01 Gew.-% gemessen an der Gesamtmasse des Stoffgemischs enthalten ist.

6. Verwendung nach einem der vorhergehenden Ansprüche, **dadurch gekennzeichnet, dass** das Lecithin in einer Menge von zwischen 1 und 2 Gew.-% gemessen an der Gesamtmasse des Stoffgemischs enthalten ist.

7. Verwendung nach einem der vorhergehenden Ansprüche, **dadurch gekennzeichnet, dass** das Stoffgemisch eines, mehrere oder alle Extrakte der Gruppe Ginseng-Extrakt, Extrakt von grünem Tee, Hamamelis-Extrakt, Kamillen-Extrakt, Kurkuma-Extrakt, Teebaum-Extrakt und Basilikum-Extrakt als Pflanzenextrakt(e) umfasst.

8. Verwendung nach Anspruch 7, **dadurch gekennzeichnet, dass** das oder die Extrakte in einer Menge von jeweils zwischen 0,2 und 0,7 Gew.-% gemessen an der Gesamtmasse des Stoffgemischs enthalten sind.

## Claims

1. Non-therapeutic use of a liquid mixture for application to the scalp, wherein the mixture comprises at least the following ingredients:
an amino acid,
Biotin,
Lecithin,
Sandalwood oil.

2. Use according to claim 1, **characterized in that** the mixture comprises arginine as an amino acid, wherein the arginine preferably is present in an amount of between 0.5 and 1.5% by weight with respect to the total mass of the mixture.

3. Use according to any one of the preceding claims, **characterized in that** the mixture comprises lysine as an amino acid, wherein the lysine preferably is present in an amount of between 0.1 and 0.4% by weight with respect to the total mass of the mixture.

4. Use according to any one of the preceding claims, **characterized in that** the mixture further comprises calcium pantothenate, wherein the calcium pantothenate is preferably present in an amount of between 0.1 and 0.5% by weight with respect to the total mass of the mixture.

5. Use according to any one of the preceding claims, **characterized in that** the biotin is present in an amount of between 0.001 and 0.01% by weight with respect to the total mass of the mixture.

6. Use according to any one of the preceding claims, **characterized in that** the lecithin is present in an amount of between 1 and 2% by weight with respect to the total mass of the mixture.

7. Use according to any one of the preceding claims, **characterized in that** the mixture comprises one, more or all of ginseng extract, green tea extract, witch hazel extract, chamomile extract, turmeric extract, tea tree extract and basil extract as plant extract(s).

8. Use according to claim 7, **characterized in that** the extract or extracts are present in an amount of between 0.2 and 0.7% by weight with respect to the total mass of the mixture.

## Revendications

1. Utilisation non thérapeutique d'un mélange de matières liquides destiné à être appliqué sur le cuir chevelu, dans laquelle le mélange de matières comprend au moins les ingrédients suivants :
un acide aminé,
de la biotine,
de la lécithine,
de l'huile de bois de santal.

2. Utilisation selon la revendication 1, **caractérisée en ce que** le mélange de matières comprend en tant qu'acide aminé de l'arginine, dans laquelle l'arginine est contenue de préférence en une quantité entre 0,5 et 1,5 % en poids mesurée par rapport à la masse totale du mélange de matières.

3. Utilisation selon l'une quelconque des revendications précédentes, **caractérisée en ce que** le mélange de matières comprend en tant qu'acide aminé de la lysine, dans laquelle la lysine est contenue de préférence en une quantité entre 0,1 et 0,4 % en poids mesurée par rapport à la masse totale du mélange de matières.

4. Utilisation selon l'une quelconque des revendications précédentes, **caractérisée en ce que** le mélange de matières comprend par ailleurs du pantothénate de calcium, dans laquelle le pantothénate de calcium est contenu de préférence en une quantité entre 0,1 et 0,5 % en poids mesurée par rapport à la masse totale du mélange de matières.

5. Utilisation selon l'une quelconque des revendications précédentes, **caractérisée en ce que** la biotine est contenue en une quantité entre 0,001 et 0,01 % en poids mesurée par rapport à la masse totale du mélange de matières.

6. Utilisation selon l'une quelconque des revendications précédentes, **caractérisée en ce que** la lécithine est contenue en une quantité entre 1 et 2 % en poids mesurée par rapport à la masse totale du mélange de matières.

7. Utilisation selon l'une quelconque des revendications précédentes, **caractérisée en ce que** le mélange de matières comprend en tant qu'extrait(s) de plante un, plusieurs ou tous les extraits du groupe extrait de ginseng, extrait de thé vert, extrait d'hamamélis, extrait de camomille, extrait de curcuma, extrait d'arbre à thé et extrait de basilic.

8. Utilisation selon la revendication 7, **caractérisée en ce que** l'extrait ou les extraits sont contenus en une quantité respectivement entre 0,2 et 0,7 % en poids mesurée par rapport à la masse totale du mélange de matières.
